(19) Europäisches Patentamt
European Patent Office
Office européen des brevets

(11) Numéro de publication: **0 057 149**
**B1**

(12) **FASCICULE DE BREVET EUROPEEN**

(45) Date de publication du fascicule du brevet: **17.04.85**

(51) Int. Cl.[4]: **C 22 C 5/06**, C 22 C 5/00

(21) Numéro de dépôt: **82400129.1**

(22) Date de dépôt: **25.01.82**

(54) **Alliages dentaires à base d'argent.**

(30) Priorité: **28.01.81 FR 8101567**

(43) Date de publication de la demande:
**04.08.82 Bulletin 82/31**

(45) Mention de la délivrance du brevet:
**17.04.85 Bulletin 85/16**

(84) Etats contractants désignés:
**BE CH DE FR GB IT LI LU NL SE**

(56) Documents cités:
**DE - C - 744 580**
**DE - C - 823 350**
**FR - A - 801 005**
**GB - A - 541 439**
**GB - A - 964 532**
**US - A - 2 572 377**

(73) Titulaire: **COMPTOIR LYON-ALEMAND - LOUYOT, 13 rue de Montmorency, F-75139 Paris Cédex 03 (FR)**

(72) Inventeur: **Niney, Claude, 20 rue Mozart, F-91470 Limours (FR)**
Inventeur: **Guerlet, Jean-Paul, 11 rue Paul Bert, F-75011 Paris (FR)**

(74) Mandataire: **Richebourg, Michel François et al, Cabinet Beau de Loménie 55, rue d'Amsterdam, F-75008 Paris (FR)**

**EP 0 057 149 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention concerne des alliages dentaires à base d'argent.

Les alliages dentaires à base d'or contiennent au moins 70% d'or. Ces alliages ne posent pas de problèmes techniques mais, pour de simples raisons de prix, on leur a parfois préféré des alliages à base d'argent.

Ces derniers sont naturellement moins chers, mais ils posent des problèmes techniques qui n'ont jamais trouvé de solution.

Il existe présentement deux grandes familles d'alliages dont l'élément prépondérant est l'argent.

Dans la présente demande, tous les pourcentages sont donnés en poids.

Parmi les alliages destinés à la réalisation de prothèses par coulée, le premier groupe |A| contient pour l'essentiel:

| | |
|---|---|
| Ag: | 30—70% |
| Pd: | 10—40% |
| Cu: | 10—20% |
| Au: | 0—30% |

On ajoute à l'argent et au palladium du cuivre pour relever les propriétés mécaniques au niveau désiré. On ajoute facultativement de l'or et, ce, jusqu'à 30% environ. La quantité d'or introduite influe directement sur la tenue à la corrosion des ces alliages en même temps que sur leur prix.

Ces alliages posent toutes sortes de problèmes techniques.

Après coulée de prothèses dentaires, l'examen micrographique montre que, quelle que soit la conception du moule, les risques de trouver un grand nombre de microporosités sont grands et il ne semble pas que l'on soit en mesure de définier des règles d'emploi qui réduiraient valablement la fréquence des défauts.

De plus, la plus grande partie des alliages de ce premier groupe posent des problèmes d'utilisation, car ils se corrodent en bouche. Cette corrosion se produit surtout avec les alliages contenant moins de 20% d'or et contenant du cuivre.

En effet, la présence de cuivre est responsable de la formation d'alliages de type biphasés, une phase étant essentiellement de l'argent additionné de palladium et éventuellement d'or, et l'autre phase étant essentiellement du cuivre additionné de palladium et éventuellement d'or. C'est la présence de cette deuxième phase qui est responsable des problèmes de corrosion.

Le problème de la corrosion en bouche peut être partiellement résolu si on choisit une teneur en or élevée. Cependant, outre que ce choix entraîne une hausse des coûts, il rend les alliages difficiles à corroyer. Aussi, un alliage contenant:

Au 25% — Ag 40% — Cu 15% — Pd 20%

ne peut être que très difficilement préparé sous forme de plots calibrés en poids ainsi qu'il convient d'opérer pour mettre à disposition des prothésistes, de la matière sous une présentation, d'emploi aisé et manipulable.

De plus, les alliages contenant beaucoup de cuivre ont tendance, lors de la coulée, à réagir avec la matière constituant le moule, ce qui confère aux pièces une surface rugueuse, oxydée, poreuse qu'il convient d'éliminer au prix d'opérations supplémentaires et au détriment de l'adaptabilité à la bouche.

La suppression du cuivre résout en partie les problèmes ci-dessus évoqués, mais en pose d'autres. Par exemple, la dureté maximale atteinte avec les alliages Ag-Pd-Au ne dépasse pas 50 HV, alors qu'il est demandé plus et que l'addition du cuivre permet d'obtenir 130 à 200 HV.

Pour contourner ces difficultés, on a essayé de remplacer le cuivre par des éléments d'addition susceptibles de relever les propriétés mécaniques des alliages Ag-Pd (avec ou sans Au) et qui n'auraient pas les inconvénients ci-dessus mentionnés.

C'est ainsi qu'on été commercialisés des alliages |B| contenant essentiellement:

| | |
|---|---|
| Pd: | 10—60% |
| Ag: | 40—70% |
| Sn: | 0—10% |
| In: | 0—10% avec Sn + In de 4 à 12% |

Dans ces alliages, l'or peut remplacer une partie de l'argent et/ou du palladium.

Paradoxalement, cette solution qui supprime le cuivre ne supprime pas ses inconvénients. Il est vrai que l'addition de Sn et/ou de In relève les propriétés mécaniques au niveau désiré comme le fait le cuivre. Par contre, les alliages contenant Sn et/ou In ont un fort retrait à la solidification et, de plus, les porosités engendrées dans ce cas sont souvent toutes localisées dans la même zone de la pièce coulée. Ceci est évidemment très défavorable et se produit de façon non contrôlable. La fréquence d'apparition de ces défauts semble ne pas dépendre des techniques utilisées.

De plus, les problèmes de tenue à la corrosion ne sont pas résolus lorsqu'on remplace du cuivre par Sn et/ou In. A quantité de métaux précieux Au et Pd contenus identiques, la sensibilité aux agents corrosifs est à peu de chose près la même.

Souvent, ces alliages, parce qu'ils ne contiennent pas de cuivre, sont utilisés pour la fabrication de prothèses composites métal-céramique.

La présence d'argent provoque un »verdissement« de la masse céramique pendant la cuisson, ce qui, en l'état des techniques, ne peut être évité.

De ceci, il ressort que les alliages de métaux précieux contenant Ag et Pd pour éléments constitutifs principaux, qu'ils contiennent Cu ou bien Sn et/ou In, ne sont jamais satisfaisants, que le fait de leur rajouter de l'or ne résout pas tous les problèmes, et que le fait de faire des alliages contenant à la fois Cu, Sn et/ou In revient à cumuler les inconvénients propres aux deux familles d'alliages |A| et |B|.

Dans le passé, il a été utilisé des alliages (groupe C) contenant:

| | |
|---|---|
| Ag: | 25—50% |
| Pd: | 40—50% |
| Pt: | 7,5—25% |

mais ces alliages ont été abandonnés notamment parce que leurs propriétés mécaniques étaient très faibles et ne pouvaient être relevées que par ajout de fortes quantités de cuivre:

Exemple:
Ag 35% — Pd 30% — Pt 10% — Au 10% — Cu 15%

ce qui nous ramène aux inconvénients déjà cités.

La présente invention permet de surmonter les diverses difficultés rencontrées lors de l'utilisation, en art dentaire, des alliages, à base d'argent.

La présente invention concerne donc de nouveaux alliages dentaires caractérisés en ce qu'ils contiennent

| | |
|---|---|
| Ag: | 20 au moins (métal de base) |
| Pd: | 7,5 à 40% |
| Pt: | 8 à 20% |
| Au: | 7,5 à 30% |

et de 2 à 10% d'au moins un métal choisi parmi le zinc, le cuivre et M, M étant le ruthénium, l'iridium et le rhodium étant entendu que

— la quantité maximale de zinc est à 5%,
— la quantité maximale de cuivre est à 8%,
— et la quantité maximale de M est à 2%.

On notera que les alliages selon l'invention ne contiennent pas d'indium ni d'étain ou alors ne contiennent que des proportions très faibles de ces métaux. De plus ces alliages ne contiennent pas de métaux suspects au plan biologique tels que cobalt, nickel, fer, chrome, manganèse ou alors contiennent moins de 0,05% de ces métaux.

Le cuivre joue, dans les alliages selon l'invention, un rôle de durcissant même lorsqu'on l'utilise en quantités relativement faibles. Cependant si la teneur en cuivre dépasse environ 6% la tenue des alliages à la corrosion a tendance à diminuer (toutes choses égales par ailleurs).

Le zinc et le métal M ont également un effet bénéfique sur les propriétés mécaniques de l'alliage et sont, en partie au moins, utilisés pour diminuer la teneur en cuivre qui apparaîtrait comme souhaitable pour l'obtention d'alliages ayant des propriétés mécaniques déterminées.

C'est dire qu'on utilisera avantageusement de façon simultanée les trois métaux Cu, Zn et M, M pouvant être lui-même un mélange de Ru, Ir, Rh, métaux qui, dans la présente invention, peuvent être considérés comme ayant des rôles équivalents.

Le platine joue un rôle important pour l'obtention d'alliages présentant de bonnes propriétés mécaniques et si la teneur en ce métal descend au-dessous d'environ 8% on assiste à une diminution notable des caractéristiques mécaniques de ces alliages.

L'or joue également un rôle non négligeable pour l'obtention de bonnes propriétés mécaniques mais de plus il a une influence très grande sur la résistance des alliages à la corrosion.

D'autres alliages selon l'invention contiennent

| | |
|---|---|
| Ag: | 30 au moins |
| Pd: | 10 à 30% de préference environ 20 |
| Pt: | 10 à 15% de préference environ 10 |
| Au: | 10 à 30% de préference entre 10 et 20 |

M: 0,05 à 1%
Cu: 0,5 à 5%
Zn: 0,5 à 2%

M ayant la même signification que précédemment et les limitations concernant les teneurs en indium et étain d'une part, cobalt, nickel, fer, chrome, manganèse d'autre partetant les mêmes que pour l'autre categorie d'alliages selon l'invention.

Les alliages selon l'invention sont beaucoup moins sujets que les alliages connus à la formation, lors de la coulée, de porosités; de plus on a pu montrer que l'on pourrait éliminer complètement ces porosités lorsque, en utilisant les alliages selon l'invention, on choisissait convenablement le nombre, le diamètre et la forme des tiges de coulée de la pièce à réaliser.

Les alliages selon l'invention ont un aspect satiné sur état brut de fonderie, exempt d'oxydation, de rugosités et autres défauts courants en fonderie à cire perdue.

Les alliages selon l'invention ont une tenue à la corrosion améliorée par rapport aux autres alliages connus, notamment lorsqu'on compare des alliages contenant des quantités identiques d'or, ou des quantités identiques d'or et de métaux de la famille du platine. Ceci est certainement dû au fait que les alliages selon l'invention ne précipitent plus une phase riche en cuivre ou en étain ou en indium. L'élément précipitant devient essentiellement le platine associé probablement à un peu de zinc, de cuivre et de ruthénium.

Ces précipités ne jouent pas défavorablement sur la tenue à la corrosion et, de façon surprenante, les faibles quantités de Ru, Cu et Zn introduites sont suffisantes pour atteindre les niveaux de caractéristiques mécaniques désirés en prothèse dentaire coulée.

Les alliages selon l'invention constituent donc une avance technique considérable tant en ce qui concerne la qualité des pièces coulées obtenues, les qualités des ajustements sur modèles, la tenue à la corrosion en milieu bucal à tel point qu'ils offrent des avantages techniques comparables a ceux offerts par les alliages dentaires dont la base est l'or, tout en offrant par rapport à ces derniers l'avantage d'une substantielle économie.

Enfin, les prothèses coulées sont polies finement avant pose en bouche de manière à éviter la fixation de la plaque dentaire.

Il a été remarqué que les alliages selon l'invention se polissent mieux, plus facilement et plus vite que les alliages à base d'argent et de palladium antérieurement connus, que ceci est probablement dû à l'aspect parfaitement sain des surfaces après coulée.

## Exemple 1

### Technique de coulée et description de certains alliages connus

Les alliages du tableau I ci-après ont été préparés sous la forme de petits lingots de 500 g environ en utilisant un four de fusion sous vide. Ils ont été homogénéisés puis laminés et découpés en plots de poids calibrés.

Avec ces plots, on a procédé à la coulée en cire perdue d'un bridge type comportant 3 éléments: canine, prémolaire et molaire avec l'élément intermédiaire plein.

Avec chaque alliage, on a procédé à plusieurs coulées au cours desquelles on a fait varier le procédé d'alimentation des pièces à couler.

- 1 tige de ∅ 2 mm par élément de bridge (A),
- 1 tige de ∅ 3 mm par élément de bridge (B),
- 1 tige de ∅ 4 mm par élément de bridge (C).

- A, puis B, puis C avec interposition d'une masselote de ∅ 5 puis 8, puis 10 sur tige correspondant à l'élément central.
- A puis B puis C avec interposition de masselotes ∅ 5 puis 8 sur les 3 tiges d'alimentation.
- Suppression de la tige d'alimentation d'un élément (D) central et alimentation par les éléments latéraux en ∅ 2 puis 3 puis 4 mm.
- D avec interposition d'une masselotte ∅ 5 puis 8 sur les 2 tiges.
- Alimentation par une seule tige sur élément central (E) en ∅ 2 puis 3 puis 4 mm.
- Montage en rateau avec tige ∅ 2 puis 3 puis 4 mm (F).
- Tous essais ci-dessus énumérés avec adjonction d'évents de dégazage sur les 3 éléments.

Après coulée, les pièces étaient coupées dans l'axe longitudinal du bridge passant par le tiges de coulée pour voir la position des porosités.

Les alliages du tableau I et ceux du même groupe comportant Sn et In qui ne sont pas mentionnés montrent tous une forte quantité de porosités qui sont en général concentrées dans une ou deux zones de la pièce coulée ce qui est évidemment dangereux.

Aucune des techniques utilisées pour la coulée ne permet de réduire les défauts ou de les localiser dans une zone choisie par avance.

Exemple 2

Les alliages du tableau II ci-après ont été préparés comme les alliages de l'exemple 1. On a procédé à la coulée d'un bridge identique a celui de l'exemple 1 et, ce, en faisant varier la méthode d'alimentation comme dans l'exemple 1.

Les bridges obtenus ont un aspect brillant après démoulage, exempt de défauts de surface et de traces d'oxydation.

Ces bridges ont été coupés selon leur axe longitudinal et préparés pour observation au microscope optique. Les porosités sont beaucoup moins nombreuses que pour les alliages de l'exemple 1. De plus, ces porosités ne sont pas concentrées dans la même zone. Elles sont en général réparties dans toute la masse ce qui est moins préjudiciable pour la tenue mécanique de la pièce coulée.

Dans ce tableau II les exemples figurant en tête sont des exemples comparatifs; l'invention est illustrée par les alliages 892 — 893 — 896 — 897 et 901.

Exemple 3

Avec les alliages du tableau II, on a procédé à la coulée d'éprouvettes de traction en respectant les prescriptions de la norme DIN 13 906.

Les éprouvettes (6 par alliage) ont été tractionnées à l'état brut de coulée. Leur dureté a été préalablement mesurée sur la partie servant de tête d'ancrage. Les résultats obtenus sont portés sur le tableau III ci-après.

On peut constater:

— que lorsque l'alliage contient moins de 5% de platine il présente des propriétés mécaniques faibles et que ces propriétés semblent meilleures pour les alliages à 10% de Pt que pour les alliages à 5% de Pt,
— qu'il est apparemment préférable que les alliages selon l'invention contiennent au moins un métal choisi parmi le Zn et M, M étant Ru, Ir ou Rh.

On a noté en outre:

— que la teneur en Zn ne doit pas être supérieure à 5% sinon les alliages montrent une vaporisation importante à la fusion,
— que lorsque la teneur en Cu dépasse 8% l'aspect de surface des objets se détériore et la teneur à la corrosion diminue.

Exemple 4

Les alliages du tableau II ont été préparés sous forme de plaquettes de 10 × 10 × 1mm, recuites au four à 800°C et refroidies lentement, puis polies avec une pâte abrasive de granulométrie égale à 1 micron.

En cet état, ils sont soumis à divers tests de corrosion comparativement à des alliages commercialisés ou à des alliages du tableau I.

Les tests de qualification comportent:

— Immersion dans $HNO_3$, d = 1,38 dilué à 5, 10 ou 20% en volume dans l'eau à 20°C, pendant 7 jours.
— Test de contact avec un coton imprégné d'une solution contenant:

| | |
|---|---|
| acide lactique: | 5% en poids |
| NaCl: | 10% en poids |
| $H_2O$ | |

a 60°C pendant 24 heures.
— Test de contact avec un coton imprégné de la solution de RINGER contenant:

| | |
|---|---|
| NaCl: | 9 g/l |
| KCl: | 0,43 g/l |
| $CaCl_2$: | 0,24 g/l |
| $NaHCO_3$: | 0,20 g/l |

pendant 72 heures à 80°C.

Les résultats sont portés dans le tableau IV ci-après.

Après 7 jours, l'alliage à 58% d'or et 5,5% de palladium est plus attaqué par l'acide $HNO_3$ à 20% que les alliages 893, 894 ou 901 qui ne contiennent que 52, 55 et 57% de métal précieux dont 22 et 20% de palladium.

L'alliage à 76% d'or n'est évidemment pas attaqué. L'alliage à 25% d'or et 20% de palladium contenant 40% d'argent et 15% de cuivre est plus attaqué que les alliages selon l'invention.

De plus, les alliages 892, 893, 894 et 901 se comportent aux tests de contact comme les alliages d'or à 58 et 70% d'or ce qui n'est pas le cas des alliages Ag-Au-Cu-Pd du commerce du groupe 1.

Les alliages du tableau I n° 008, 803, 402 et 874 n'ont pas une tenue à $HNO_3$ meilleure que les alliages Ag-Au-Cu-Pd du groupe 1.

Tableau I (Exemples comparatifs)

| Alliage | Composition % | | | | | | | | Intervalle |
|---|---|---|---|---|---|---|---|---|---|
| | Ag | Pd | Pt | Cu | Zn | Au | Sn | Divers | de fusion °C |
| 008 | 69 | 7 | | | | 20 | 4 | | 949—992 |
| 009 | 66 | 7 | | | 3 | 20 | 4 | | 865—950 |
| 081 | 67 | 7 | 2 | | | 20 | 4 | | |
| 836 | 67,2 | 7 | 15 | | | 20 | 4 | Fe 0,3 | 945—990 |
| 824 | 66 | 20 | | | | 10 | 4 | | 1018—1064 |
| 012 | 67 | 20 | 9 | | | | 4 | | 1032—1097 |
| 015 | 80 | | 15 | | 1 | | 4 | | 890—960 |
| 017 | 79 | | 10 | | | 7 | 4 | | 912—970 |
| 021 | 75 | | 5 | | 1 | 15 | 4 | | 882—951 |
| 151 | 72 | 10 | 13 | | 1 | | 4 | | 977—1016 |
| 181 | 71 | 10 | 9 | | 1 | 5 | 4 | | 969—1008 |
| 402 | 63 | 10 | | 3 | 2 | 20 | 2 | | 824—922 |
| 405 | 65 | 10 | | 3 | 2 | 20 | | | 873—944 |
| 408 | 65 | 10 | | 3 | | 20 | 2 | | 881—948 |
| 411 | 67 | 10 | | 3 | | 20 | | | 929—972 |
| 402/B | 58 | 15 | 2 | 3 | 2 | 18 | 2 | | 949—1006 |
| 803 | 46,8 | 40 | 2 | | | 5 | 6 | Ir 2 | |
| 873 | 69 | | 7 | | | 20 | 4 | | |
| 874 | 62 | 7 | 7 | | | 20 | 4 | | |
| 876 | 62 | 4 | 7 | 3 | | 20 | 4 | | |

Tableau II

| Alliage | Composition % Ag | Pd | Pt | Ru | Cu | Zn | Au |
|---|---|---|---|---|---|---|---|
| 881 | 56 | 30 | 5 | | 4 | | 5 |
| 882 | 56 | 30 | 10 | | 4 | | |
| 883 | 69 | 20 | 2 | | 4 | | 5 |
| 884 | 64 | 22 | 5 | | 4 | | 5 |
| 887 | 61 | 22 | 10 | 1 | 4 | 2 | |
| 890 | 65,5 | 22 | 10 | 0,5 | | 2 | |
| 892 | 53,5 | 22 | 10 | 0,5 | 2 | 2 | 10 |
| 893 | 43,5 | 22 | 10 | 0,5 | 2 | 2 | 20 |
| 896 | 50,5 | 22 | 10 | 0,5 | 6 | 1 | 10 |
| 897 | 48,5 | 22 | 10 | 0,5 | 8 | 1 | 10 |
| 901 | 38 | 20 | 10 | | 4 | 1 | 27 |

Tableau III

Caractéristiques mécaniques

| Alliage | Dureté Vickers daN/mm$^2$ | Résistance mécanique R (daN/mm$^2$) | Résistance élastique $E_{0,2}$ (daN/mm$^2$) | Allongement % |
|---|---|---|---|---|
| 881 | 94,9 | 33,8 | 18,9 | 35,8 |
| 882 | 113,1 | 37,5 | 22,8 | 26,2 |
| 883 | 85,3 | 28,7 | 18,1 | 34,1 |
| 884 | 98,1 | 33,2 | 18,1 | 32,8 |
| 887 | 139,1 | 46,9 | 29,0 | 22,5 |
| 890 | 121,3 | 39,3 | 25,9 | 21,4 |
| 892 | 144,7 | 48,4 | 30,4 | 23,9 |
| 893 | 166,0 | 49,2 | 34,9 | 15,4 |
| 896 | 164,5 | 51,3 | 37 | 18 |
| 897 | 167 | 52,9 | 37,5 | 17,5 |
| 901 | 137 | 47,4 | 28,7 | 24,2 |

Tableau IV

| Alliage | Pd+Pt | Au | Test $HNO_3$ 5% (1) | Test $HNO_3$ 10% (1) | Test $HNO_3$ 20% (1) | Test acide lactique +NaCl (2) | Test RINGER (2) |
|---|---|---|---|---|---|---|---|
| 881 | 35 | 5 | Néant | $11,2 \cdot 10^{-4}$ | | Attaque faible | Pas d'attaque |
| 882 | 40 | | Néant | Néant | | Attaqué | Pas d'attaque |
| 883 | 22 | 5 | Néant | $49,2 \cdot 10^{-4}$ | | Attaque faible | Pas d'attaque |
| 884 | 27 | 5 | $58,3 \cdot 10^{-5}$ | 14,5 | | Attaqué | Pas d'attaque |
| 887 | 32 | | $41,2 \cdot 10^{-5}$ | $2,3 \cdot 10^{-4}$ | | Attaqué | Pas d'attaque |
| 890 | 32 | | Néant | $33,5 \cdot 10^{-4}$ | | Attaqué | Pas d'attaque |
| 892 | 32 | 10 | Néant | $4,31 \cdot 10^{-4}$ | $12,3 \cdot 10^{-3}$ | Non attaqué | Pas d'attaque |
| 893 | 32 | 20 | Néant | Néant | $0,4 \cdot 10^{-3}$ | Non attaqué | Pas d'attaque |
| 896 | 32 | 10 | Néant | $18 \cdot 10^{-4}$ | $24,5 \cdot 10^{-3}$ | Non attaqué | Pas d'attaque |
| 897 | 32 | 10 | Néant | $10,7 \cdot 10^{-4}$ | $54,5 \cdot 10^{-3}$ | Non attaqué | Pas d'attaque |
| 901 | 30 | 27 | Néant | Néant | $0,2 \cdot 10^{-3}$ | Non attaqué | Pas d'attaque |
| 008 | 7 | 20 | | $53,4 \cdot 10^{-4}$ | $98,4 \cdot 10^{-3}$ | Faible attaque | Pas d'attaque |
| 803 | 42 | 5 | | $15,2 \cdot 10^{-4}$ | $24,4 \cdot 10^{-3}$ | Faible attaque | Pas d'attaque |
| 402 | 10 | 20 | | $33,1 \cdot 10^{-4}$ | $14,2 \cdot 10^{-3}$ | Attaqué | Pas d'attaque |
| 874 | 14 | 20 | | $3,9 \cdot 10^{-4}$ | | Faible attaque | Pas d'attaque |

Tableau IV (suite)

| Alliage | Pd+Pt | Au | Test $HNO_3$ 5% (1) | Test $HNO_3$ 10% (1) | Test $HNO_3$ 20% (1) | Test acide lactique +NaCl (2) | Test RINGER (2) |
|---|---|---|---|---|---|---|---|
| Ag 55, Cu 15 Au 10, Pd 20 | 20 | 10 | $66,9 \cdot 10^{-5}$ | $14,3 \cdot 10^{-4}$ | *) | Attaqué | Pas d'attaque |
| Ag 40, Cu 15 Au 25, Pd 20 | 20 | 25 | Néant | Néant | $3,6 \cdot 10^{-3}$ | Attaque faible | Pas d'attaque |
| Au 58, Ag 23 Pd 5,5, Cu 12 Zn 1,5 | 5,5 | 58 | | | $1,5 \cdot 10^{-3}$ | Pas d'attaque | Pas d'attaque |
| Au 76, Ag 10 Cu 10, Pt 2,5 Pd 1,5 | 4 | 76 | | | Néant | Pas d'attaque | Pas d'attaque |

*) Totalement détruit.
(1) Pertes de poids en g par g après 7 jours.
(2) Inspection en microscopie optique.

**Revendications**

1. Alliages dentaires, caractérisés en ce qu'ils contiennent:

| | |
|---|---|
| Ag: | 20 au moins (métal de base) |
| Pd: | 7,5 à 40% |
| Pt: | 8 à 20% |
| Au: | 7,5 à 30% |

et de 2 à 10% d'au moins un métal choisi parmi le zinc, le cuivre et M., M étant le ruthénium, l'iridium et le rhodium, étant entendu que:

- les quantités maximales de zinc, cuivre et M sont respectivement 5%, 8% et 2%,
- les alliages ne contiennent ni indium, ni étain et moins de 0,05% de métaux allergènes tels que fer, cobalt, nickel, chrome, manganèse.

2. Alliages dentaires caractérisés en ce qu'ils contiennent:

| | |
|---|---|
| Ag: | 30 au moins |
| Pd: | 10 à 30% |
| Pt: | 10 à 15% |
| Au: | 10 à 30% |
| M: | 0,05 à 1% |
| Cu: | 0,5 à 5% |
| Zn: | 0,5 à 2% |

M étant le ruthénium, l'iridium et le rhodium, étant entendu que:

- les quantités maximales de zinc, cuivre et M sont respectivement 5%, 8% et 2%,
- les alliages ne contiennent ni indium, ni étain et moins de 0,05% de métaux allergènes tels que fer, cobalt, nickel, chrome, manganèse.

**Patentansprüche**

1. Zahnlegierungen, dadurch gekennzeichnet, daß sie folgendes enthalten:

| | |
|---|---|
| Ag: | mindestens 20% (Basismetall) |
| Pd: | 7,5 bis 40% |
| Pt: | 8 bis 20% |
| Au: | 7,5 bis 30% |

und 2 bis 10% von zumindest einem Metall, ausgewählt aus Zink, Kupfer und M, wobei M Ruthenium, Iridium und Rhodium bedeutet, mit der Maßgabe, daß

- die Maximalmengen von Zink, Kupfer und M 5%, 8% bzw. 3% betragen,
- die Legierungen weder Indium noch Zinn und weniger als 0,05% von allergenen Metallen, wie Eisen, Kobalt, Nickel, Chrom, Mangan, enthalten.

2. Zahnlegierungen, dadurch gekennzeichnet, daß sie folgendes enthalten:

| | |
|---|---|
| Ag: | mindestens 30% |
| Pd: | 10 bis 30% |
| Pt: | 10 bis 15% |
| Au: | 10 bis 30% |
| M: | 0,05 bis 1% |
| Cu: | 0,5 bis 5% |
| Zn: | 0,5 bis 2% |

wobei M Ruthenium, Iridium und Rhodium bedeutet, mit der Maßgabe, daß

- die Maximalmengen von Zink, Kupfer und M 5%, 8% bzw. 2% betragen,
- die Legierungen weder Indium noch Zinn und weniger als 0,05% von allergenen Metallen, wie Eisen, Kobalt, Nickel, Chrom, Mangan, enthalten.

**Claims**

1. Dental alloys, characterized in that they contain:

| | |
|---|---|
| Silver | 20 at least (basic metall) |
| Palladium | 7.5 to 40% |
| Platinum | 8 to 20% |
| Gold | 7.5 to 30% |

and from 2 to 10% at least of a metal selected from zinc, copper, and M, M being ruthenium, iridium and rhodium, on the understanding that:

— the maximal quantities of zinc, copper and M are respectively 5%, 8% and 2%,
— the alloys contain no indium, tin and less than 0.05% of allergen metals such as iron, cobalt, nickel, chromium, manganese.

2. Dental alloys, characterized in that they contain:

| | |
|---|---|
| Silver | 30 at least |
| Palladium | 10 to 30% |
| Platinum | 10 to 15% |
| Gold | 10 to 30% |
| M | 0.05 to 1% |
| Copper | 0.5 to 5% |
| Zinc | 0.5 to 2% |

M being ruthenium, iridium and rhodium, on the understanding that:

— the maximal quantities of zinc, copper and M are respectively 5%, 8% and 2%,
— the alloys contain no indium, no tin and less than 0.05% of allergen metals such as iron, nickel, chromium, manganese.